(19) Europäisches Patentamt
European Patent Office
Office européen des brevets

(11) Veröffentlichungsnummer: **0 117 485**
**A2**

# (12) EUROPÄISCHE PATENTANMELDUNG

(21) Anmeldenummer: **84101587.8**

(22) Anmeldetag: **16.02.84**

(51) Int. Cl.³: **C 07 D 213/30**, C 07 D 213/70, C 07 D 213/32, C 07 D 213/26, C 07 D 241/12, C 07 D 239/26, C 07 D 405/06, C 07 D 409/06, A 01 N 43/40, A 01 N 43/54, A 01 N 43/10 // C07D213/50

(30) Priorität: **28.02.83 CH 1083/83**
**02.12.83 CH 6463/83**

(43) Veröffentlichungstag der Anmeldung: **05.09.84**
**Patentblatt 84/36**

(84) Benannte Vertragsstaaten: **AT BE CH DE FR GB IT LI NL**

(71) Anmelder: **F. HOFFMANN-LA ROCHE & CO.**
**Aktiengesellschaft, CH-4002 Basel (CH)**

(72) Erfinder: **Dorn, Franz, Dr., Bohnackerstrasse 5,**
**CH-8157 Dielsdorf (CH)**

(74) Vertreter: **Lederer, Franz, Dr. et al, Patentanwälte Dr. Franz Lederer Dipl.-Ing. Reiner F. Meyer-Roxlau**
**Lucile-Grahn-Strasse 22, D-8000 München 80 (DE)**

## (54) Pyridin-, Pyrazin- und Pyrimidinderivate und deren Verwendung als fungizide Wirkstoffe.

(57) Die Erfindung betrifft neue Pyridin-, Pyrazin- und Pyrimidinderivate der Formel

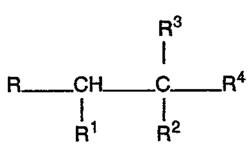

I

worin R, $R^1$, $R^2$, $R^3$ und $R^4$ die in der Beschreibung angegebenen Bedeutungen besitzen, und ihre Säureadditionssalze, Verfahren zu deren Herstellung, fungizide Mittel, die diese Verbindungen als Wirkstoffe enthalten sowie die Verwendung solcher Verbindungen bzw. Mittel zur Bekämpfung von Fungi in der Landwirtschaft und im Gartenbau. Die Erfindung betrifft ferner bei der Herstellung von Verbindungen der Formel I verwendbare Mittel.

EP 0 117 485 A2

F.Hoffmann-La Roche & Co.Aktiengesellschaft, Basel/Schweiz 0117485

1 0. Feb. 1984

RAN 6103/26



# Pyridin-, Pyrazin- und Pyrimidinderivate und deren Verwendung als fungizide Wirkstoffe

Die Erfindung betrifft heterocyclische Verbindungen, und zwar Pyridin-, Pyrazin- und Pyrimidinderivate der allgemeinen Formel

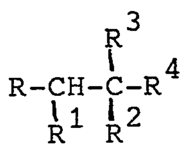

I

worin R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ gegebenenfalls mit 1 bis 3 Halogenatomen substituiertes $C_{3-6}$-Alkenyl, $C_{3-6}$-Alkinyl, $C_{3-6}$-Alkenyloxy, $C_{3-6}$-Alkinyloxy, $C_{3-6}$-Alkenylthio oder $C_{3-6}$-Alkinylthio; oder gegebenenfalls im Arylkern mono-, di- oder trisubstituiertes Aryl-$C_{1-3}$-alkyl, Aryloxy oder Arylthio, wobei die gegebenenfalls vorhandenen Substituenten 1 bis 3 Halogenatome und/oder 1 oder 2 $C_{1-3}$-Alkylgruppen

und/oder 1 oder 2 $C_{1-3}$-Alkoxygruppen und/oder eine Nitrogruppe sind,

$R^2$ Hydroxy, Chlor oder Brom,

$R^3$ Wasserstoff oder $C_{1-4}$-Alkyl

und $R^4$ 3-Pyridyl, 3-Pyridyl-1-oxid, 2-Pyrazinyl, 2-Pyrazinyl-1-oxid, 2-Pyrazinyl-4-oxid, 2-Pyrazinyl-1,4-dioxid, 5-Pyrimidinyl, 5-Pyrimidinyl-1-oxid oder 5-Pyrimidinyl-1,3-dioxid bedeuten,

und Säureadditionssalze dieser Verbindungen.

Die Verbindungen der Formel I und deren Säureadditionssalze besitzen fungizide Eigenschaften und eignen sich als fungizide Wirkstoffe, insbesondere zur Verwendung in der Landwirtschaft und im Gartenbau.

Die Erfindung betrifft ferner ein Verfahren zur Herstellung der Verbindungen der Formel I und ihrer Säureadditionssalze, Verbindungen der Formel I und Säureadditionssalze davon als fungizide Wirkstoffe, fungizide Mittel, die Verbindungen der Formel I oder Säureadditionssalze davon als Wirkstoffe enthalten, sowie die Verwendung solcher Verbindungen, Säureadditionssalze und Mittel zur Bekämpfung von Fungi in der Landwirtschaft und im Gartenbau.

In der obigen Formel I umfasst der Ausdruck "Halogen" Fluor, Chlor, Brom und Jod. Die Alkyl- , Alkenyl- und Alkinylgruppen können geradkettig oder verzweigt sein, wobei dies auch für den Alkyl-, Alkenyl- oder Alkinylteil der Alkoxy-, Arylalkyl-, Alkenyloxy-, Alkenylthio-, Alkinyloxy- und Alkinylthiogruppen gilt. Der Ausdruck "Aryl" an sich oder als Teil von "Aryloxy" oder "Arylthio" bedeutet vorzugsweise Phenyl, wobei aber auch heterocyclische Gruppen mit aromatischem Charakter, wie Pyridyl, Furyl und Thienyl, in Betracht kommen. In di- oder trisubstituiertem Phenyl, Alkenyl, Alkinyl, Alkenyloxy, Alkinyloxy, Alkenylthio, Alkinylthio, Arylalkyl, Aryloxy oder Arylthio können die Substituenten gleich oder verschieden sein.

Im allgemeinen ist die Alkenylgruppe vorzugsweise Allyl und die Alkinylgruppe vorzugsweise Propargyl.

Unabhängig voneinander bedeuten R vorzugsweise Mono-, Di- oder Trihalophenyl, insbesondere 2,4-Dichlorphenyl; $R^1$ vorzugsweise gegebenenfalls substituiertes Alkenyl, Alkinyl, Alkenylthio, Alkinylthio, Arylalkyl oder Arylthio, insbesondere gegebenenfalls substituiertes Alkenyl, Alkinyl oder Arylthio, wie diese Reste oben näher definiert sind; $R^3$ vorzugsweise Wasserstoff oder Methyl; und $R^4$ vorzugsweise 3-Pyridyl, 2-Pyrazinyl oder 5-Pyrimidinyl.

Besonders bevorzugte Verbindungen der Formel I sind:

α-(α-Allyl-2,4-dichlorbenzyl)-3-pyridylmethanol,
α-[2,4-Dichlor-α-(2-propinyl)-benzyl]-3-pyridylmethanol,
α-[α-(2,4-Dichlorphenyl)-phenäthyl]-3-pyridylmethanol und
α-(2,4-Dichlor-α-phenylthio-benzyl)-3-pyridylmethanol.

Weitere Vertreter von Verbindungen der Formel I sind:

α-[2,4-Dichlor-α-phenoxy-benzyl]-α-methyl-3-pyridylmethanol,
α-[α-Allyloxy-2,4-dichlorbenzyl]-α-methyl-3-pyridylmethanol,
α-[2,4-Dichlor-α-propargyloxy-benzyl]-α-methyl-3-pyridylmethanol,
α-[α-Allyloxy-2,4-dichlorbenzyl]-3-pyridylmethanol,
α-[α-Allyloxy-2,4-dichlorbenzyl]-3-pyridylmethylchlorid,
α-[α-Allylthio-2,4-dichlorbenzyl]-3-pyridylmethanol und

α-[α-Allyloxy-2,4-dichlorbenzyl]-α-methyl-2-pyrazinylmethanol.

Das Vorhandensein von mindestens zwei asymmetrischen Kohlenstoffatomen in den Verbindungen der Formel I hat zur Folge, dass die Verbindungen in isomeren Formen auftreten können, welche in enantiomerer oder diastereomerer Beziehung zueinander stehen. Durch das Vorliegen einer allfälligen aliphatischen C=C-Doppelbindung kann auch geometrische Isomerie auftreten. Die Formel I soll all diese möglichen isomeren Formen umfassen.

Als Säureadditionssalze der Verbindungen der Formel I kommen physiologisch verträgliche Salze in Frage. Hierzu gehören vorzugsweise Salze dieser Verbindungen mit anorganischen und organischen Säuren, wie Salzsäure; Salpetersäure; Phosphorsäure; mono- und bifunktionellen Carbonsäuren und Hydroxycarbonsäuren, z.B. Essigsäure, Maleinsäure, Bernsteinsäure, Fumarsäure, Weinsäure, Zitronensäure, Salicylsäure, Sorbinsäure und Milchsäure; und Sulfonsäuren, z.B. 1,5-Naphthalin-disulfonsäure. Im Falle der N-Oxide, d.h. der Verbindungen der Formel I, worin $R^2$ 3-Pyridyl-1-oxid, 2-Pyrazinyl-1-oxid, -4-oxid und -1,4-dioxid, und 5-Pyrimidinyl-1-oxid und -1,3-dioxid, kommen als Säureadditionssalze insbesondere physiologisch verträgliche Salze mit starken Säuren in Frage, wie anorganischen Säuren, z.B. Salzsäure, Salpetersäure und Phosphorsäure, und Sulfonsäuren, z.B. 1,5-Naphthalindisulfonsäure.

Das erfindungsgemässe Verfahren zur Herstellung der Verbindungen der Formel I und von ihren Säureadditionssalzen ist dadurch gekennzeichnet, dass man

a) zwecks Herstellung der Verbindungen der Formel I, worin $R^1$ gegebenenfalls substituiertes $C_{3-6}$-Alkenyloxy, $C_{3-6}$-Alkinyloxy oder Aryloxy, wie oben näher definiert, $R^2$ Hydroxy und $R^4$ 3-Pyridyl, 2-Pyrazinyl oder 5-Pyrimidinyl bedeuten, ein Epoxid der allgemeinen Formel

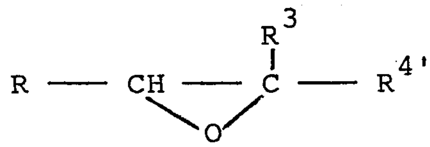 II

worin R und $R^3$ die oben angegebenen Bedeutungen besitzen

und $R^{4'}$ 3-Pyridyl, 2-Pyrazinyl oder 5-Pyrimidinyl bedeutet,

oder einen Alkohol der allgemeinen Formel

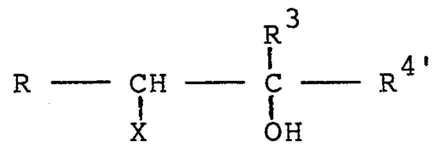 III

worin R, $R^3$ und $R^{4'}$ die oben angegebenen Bedeutungen besitzen

und X ein Halogenatom, z.B. Chlor oder Brom, einen Alkylsulfonatrest, z.B. den Methansulfonatrest, oder einen Arylsulfonatrest, z.B. den Benzolsulfonat- oder p-Toluolsulfonatrest, bedeutet,

mit einer Verbindung der allgemeinen Formel

$$R^{1'} — H \qquad IV$$

worin $R^{1'}$ gegebenenfalls mit 1 bis 3 Halogenatomen substituiertes $C_{3-6}$-Alkenyloxy oder $C_{3-6}$-Alkinyloxy; oder gegebenenfalls mono-, di- oder trisubstituiertes Aryloxy, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind, bedeutet,

umsetzt,

b) zwecks Herstellung der Verbindungen der Formel I, worin $R^1$ gegebenenfalls substituiertes $C_{3-6}$-Alkenyl, $C_{3-6}$-Alkinyl, $C_{3-6}$-Alkenylthio, $C_{3-6}$-Alkinylthio, Aryl-$C_{1-3}$-alkyl oder Arylthio, wie oben näher definiert, $R^2$ Hydroxy, $R^3$ Wasserstoff und $R^4$ 3-Pyridyl, 2-Pyrazinyl oder 5-Pyrimidinyl bedeuten, ein Keton der allgemeinen Formel

$$R - \underset{R^{1''}}{\underset{|}{CH}} - \underset{O}{\underset{\|}{C}} - R^{4'} \qquad V$$

worin R und $R^{4'}$ die oben angegebenen Bedeutungen besitzen

und $R^{1''}$ gegebenenfalls mit 1 bis 3 Halogenatomen substituiertes $C_{3-6}$-Alkenyl, $C_{3-6}$-Alkinyl, $C_{3-6}$-Alkenylthio oder $C_{3-6}$-Alkinylthio; oder gegebenenfalls im Arylkern mono-, di- oder trisubstituiertes Aryl-$C_{1-3}$-alkyl oder Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind, bedeutet,

reduziert,

c) zwecks Herstellung der Verbindungen der Formel I, worin $R^1$ gegebenenfalls substituiertes $C_{3-6}$-Alkenyl, $C_{3-6}$-Alkinyl, $C_{3-6}$-Alkenylthio, $C_{3-6}$-Alkinylthio, Aryl-$C_{1-3}$-alkyl oder Arylthio, wie oben näher definiert, $R^2$ Hydroxy, $R^3$ $C_{1-4}$-Alkyl und $R^4$ 3-Pyridyl, 2-Pyrazinyl oder 5-Pyrimidinyl bedeuten, ein Keton der allgemeinen Formel V, wie oben definiert, mit einer Verbindung der allgemeinen Formel

$$R^{3'} - Y \qquad VI$$

worin $R^{3'}$ $C_{1-4}$-Alkyl,

Y Lithium oder MgZ

und Z Halogen, insbesondere Brom oder Jod, bedeuten,

umsetzt,

d) zwecks Herstellung der Verbindungen der Formel I, worin $R^2$ Chlor oder Brom und $R^4$ 3-Pyridyl, 2-Pyrazinyl oder 5-Pyrimidinyl bedeuten, einen Alkohol der allgemeinen Formel

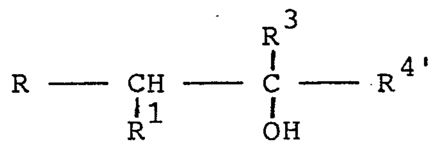

I'

worin R, $R^1$, $R^3$ und $R^{4'}$ die oben angegebenen Bedeutungen besitzen,

mit einem Chlorierungsmittel bzw. Bromierungsmittel behandelt,

e) zwecks Herstellung der Verbindungen der Formel I, worin $R^1$ gegebenenfalls substituiertes $C_{3-6}$-Alkenylthio, $C_{3-6}$-Alkinylthio oder Arylthio, wie oben näher definiert, und $R^2$ Hydroxy bedeuten, einen Thioäther der allgemeinen Formel

$$R - CH_2 - R^{1'''} \qquad VII$$

worin R die oben angegebene Bedeutung besitzt und $R^{1'''}$ gegebenenfalls mit 1 bis 3 Halogenatomen substituiertes $C_{3-6}$-Alkenylthio oder $C_{3-6}$-Alkinylthio; oder gegebenenfalls mono-, di- oder trisubstituiertes Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind, bedeutet,

mit einem Aldehyd bzw. Keton der allgemeinen Formel

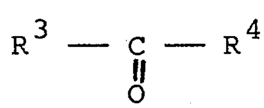

VIII

worin $R^3$ und $R^4$ die oben angegebenen Bedeutungen besitzen,

umsetzt, oder

f) zwecks Herstellung der Verbindungen der Formel I, worin $R^4$ 3-Pyridyl-1-oxid, 2-Pyrazinyl-1-oxid, 2-Pyrazinyl-4-oxid, 2-Pyrazinyl-1,4-dioxid, 5-Pyrimidinyl-1-oxid oder 5-Pyrimidinyl-1,3-dioxid bedeutet, ein Pyridin-, Pyrazin- bzw. Pyrimidinderivat der allgemeinen Formel

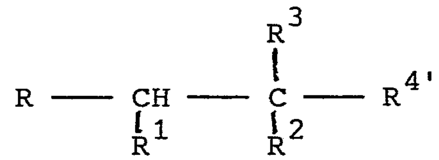

I"

worin R, $R^1$, $R^2$, $R^3$ und $R^{4'}$ die oben angegebenen Bedeutungen besitzen,

N-oxydiert

und erwünschtenfalls eine erhaltene Verbindung der Formel I durch Umsetzung mit einer Säure in das entsprechende Säureadditionssalz überführt.

Bei der Verfahrensvariante a) wird zweckmässigerweise mit einem Ueberschuss der Verbindung der Formel IV und in einem Temperaturbereich zwischen 0°C und der Rückflusstemperatur des Reaktionsgemisches gearbeitet. Im Falle der Umsetzung mit dem Epoxid der Formel II beträgt der bevorzugte Temperaturbereich 0°C bis 70°C und der bevorzugte Ueberschuss der Verbindung der Formel IV bis zu 200 Gewichtsprozent. Man arbeitet im Falle der Umsetzung mit dem Alkohol der Formel III vorzugsweise in einem Temperaturbereich zwischen der Raumtemperatur und der Rückflusstemperatur des Reaktionsgemisches. Im Falle der Umsetzung des Alkohols III mit der Verbindung IV kann die letztere auch als Metallsalz, vorzugsweise als Alkalimetall- oder Erdalkalimetallsalz, eingesetzt werden.

Zudem kann in beiden Fällen die Umsetzung in einem Verdünnungsmittel erfolgen. Wird ein Verdünnungsmittel benützt, so ist dies vorzugsweise das im Ueberschuss verwendete Reagens der Formel IV oder ein inertes organisches Lösungsmittel, wie ein aromatischer Kohlenwasserstoff, z.B. Benzol oder Toluol; ein aliphatischer oder cyclischer Aether, z.B. Diäthyläther, Dioxan oder Tetrahydrofuran; ein halogenierter aliphatischer Kohlenwasserstoff, z.B. Methylenchlorid, Chloroform oder Tetrachlorkohlenstoff; oder Dimethylformamid.

Für die Reaktion mit dem Epoxid der Formel II ist oft die Zugabe einer Mineralsäure, z.B. Schwefelsäure, einer Sulfonsäure, z.B. p-Toluolsulfonsäure, oder einer Lewis-Säure, z.B. Aluminiumchlorid, Zinkchlorid oder Bortrifluorid, von Vorteil.

Bei der Umsetzung mit dem Alkohol der Formel III resultiert eine Inversion am die Gruppen R und X tragenden Kohlenstoffatom, so dass z.B. aus einem Alkohol der Formel III, eine andere enantiomere Form des Endproduktes der Formel I hergestellt werden kann als aus dem entsprechenden Epoxid der Formel II.

Beispiele von in dieser Verfahrensvariante gegebenenfalls verwendeten Salzen der Verbindung der Formel IV sind Alkalimetallsalze, wie die Natrium- und Kaliumsalze, und Erdalkalimetallsalze, wie die Calcium- und Magnesiumsalze.

Es erweist sich als vorteilhaft, den Alkohol der Formel III mittels Umsetzung des entsprechenden Epoxids der Formel II mit einer Alkyl- bzw. Arylsulfonsäure oder mit Chlorwasserstoff bzw. Bromwasserstoff in situ herzustellen und anschliessend den resultierenden Alkohol der Formel III ohne Isolierung mit einer Verbindung der Formel IV oder einem Salz davon zum Endprodukt umzusetzen.

Die Reduktion gemäss Verfahrensvariante b) erfolgt vorzugsweise mittels eines komplexen Metallhydrids, wie

Natriumborhydrid, wobei in einem protischen Verdünnungsmittel, wie einem Alkohol, z.B. Methanol oder Aethanol, bei Temperaturen um Raumtemperatur gearbeitet wird, oder Lithiumaluminiumhydrid, das in einem aprotischen Verdünnungsmittel, insbesondere einem aliphatischen oder cyclischen Aether, z.B. Diäthyläther, Tetrahydrofuran oder Dioxan, bei Reaktionstemperaturen zwischen 0°C und der Raumtemperatur eingesetzt wird. Als komplexe Metallhydride in geeigneten Lösungsmitteln kommen auch Lithiumborhydrid in Aethanol oder Tetrahydrofuran, Natriumborhydrid-aluminiumchlorid in einem Aether, z.B. Diglym, und Lithiumtri-(tert. butoxy)aluminiumhydrid in Tetrahydrofuran in Frage. Das Keton der Formel V kann auch z.B. mittels Diboran in Tetrahydrofuran oder mittels an sich bekannter katalytischer Hydrierung zur Verbindung der Formel I reduziert werden. Die Reaktionsbedingungen sind dem Fachmann aus analogen Reduktionen geläufig.

Die Verfahrensvariante c) wird zweckmässigerweise durchgeführt, indem man das Keton der Formel V mit der Verbindung der Formel VI in einem inerten Verdünnungsmittel, vorzugsweise einem aprotischen Lösungsmittel, wie einem aliphatischen oder cyclischen Aether, z.B. Diäthyläther, Tetrahydrofuran oder Dioxan, und in einem Temperaturbereich zwischen -70°C und der Rückflusstemperatur des Reaktionsgemisches, vorzugsweise zwischen -30°C und der Raumtemperatur, umsetzt.

Die Umsetzung des Alkohols der Formel I' nach Verfahrensvariante d) wird vorzugsweise unter Verwendung von Phosphorpentachlorid, Thionylchlorid oder Phosphoroxychlorid als Chlorierungsmittel bzw. von Phosphortribromid als Bromierungsmittel durchgeführt. Bei der Durchführung wird zweckmässigerweise in Gegenwart eines inerten Verdünnungsmittels, insbesondere eines aprotischen organischen Lösungsmittels, und gegebenenfalls auch in Gegenwart einer Base gearbeitet. Zu den bevorzugten Verdünnungsmitteln gehören aliphatische und aromatische Kohlenwasserstoffe, wie

n-Hexan, Benzol, Toluol und Xylole; halogenierte aliphatische Kohlenwasserstoffe, wie Methylenchlorid, Chloroform und Tetrachlorkohlenstoff; halogenierte aromatische Kohlenwasserstoffe, wie Chlorbenzol; und tertiäre Amine, wie Triäthylamin und Pyridin. Bevorzugte Basen sind Triäthylamin, Pyridin und Calciumcarbonat. Die Reaktionstemperaturen liegen im allgemeinen zwischen 0°C und der Rückflusstemperatur des Reaktionsgemisches, vorzugsweise zwischen der Raumtemperatur und der Rückflusstemperatur. Das Chlorierungsmittel bzw. Bromierungsmittel wird vorzugsweise im Ueberschuss verwendet.

Bei der Verfahrensvariante e) wird zweckmässigerweise der Thioäther der Formel VII zuerst mit einer starken Base, wie einem Alkalimetallamid, z.B. Lithiumdiisopropylamid, in einem inerten Verdünnungsmittel, wie einem Kohlenwasserstoff oder einem aliphatischen oder cyclischen Aether, z.B. Tetrahydrofuran, und bei einer Temperatur von ca. -20°C behandelt. Der Zusatz eines Komplexierungsmittels, z.B. Hexamethylphosphorsäuretriamid, zum Reaktionsgemisch erweist sich als vorteilhaft. Anschliessend wird der Aldehyd bzw. das Keton der Formel VIII zugefügt und das Reaktionsgemisch auf Raumtemperatur gebracht.Auf diese Weise wird die Reaktion normalerweise innert kurzer Zeit beendet.

Bei der Verfahrensvariante f) handelt es sich um die N-Oxydation derjenigen Endprodukte der Formel I, worin $R^4$ die oben angegebenen Bedeutungen von $R^{4'}$ besitzt. Die Umsetzung kann zweckmässigerweise durchgeführt werden, indem man die Verbindung der Formel I" mittels Wasserstoffperoxid oder einer Persäure in Gegenwart eines inerten Verdünnungsmittel N-oxydiert.

Im Falle der Verwendung von Wasserstoffperoxid als Oxydationsmittel kommen als Verdünnungsmittel insbesondere niedere Alkanole, wie Methanol, Aethanol und Isopropanol, in Frage, und die N-Oxydation wird vorzugsweise in einem Temperaturbereich zwischen 0° und 60°C, insbesondere zwischen 20° und 40°C, vorgenommen.

Als Persäuren kommen vorzugsweise Peressigsäure, Perbenzoesäure und m-Chlorperbenzoesäure in Frage, wobei bevorzugt in einem halogenierten Kohlenwasserstoff, z.B. Methylenchlorid oder Chloroform, als Verdünnungsmittel gearbeitet wird. Die N-Oxydation mit einer Persäure erfolgt vorzugsweise in einem Temperaturbereich zwischen 0°C und der Rückflusstemperatur des Reaktionsgemisches, insbesondere zwischen 0°C und der Raumtemperatur. Eine besonders bevorzugte Ausführungsform dieses Verfahrens besteht darin, dass man die N-Oxydation mit m-Chlorperbenzoesäure in Chloroform in einem Temperaturbereich zwischen 0°C und der Raumtemperatur durchführt.

Zur Herstellung der Säureadditionssalze der Verbindungen der Formel I werden die Verbindungen I mit den gewünschten Säuren auf übliche Weise umgesetzt.

Die Isolierung und Reinigung der so hergestellten Verbindungen der Formel I bzw. der Säureadditionssalze können nach an sich bekannten Methoden erfolgen.

Die als Ausgangsmaterialien verwendbaren Epoxide der allgemeinen Formel II sind grösstenteils neu. Sie können hergestellt werden, indem man ein Halogenid der allgemeinen Formel

$$R — CH_2 — Hal \qquad IX$$

worin R die oben angegebene Bedeutung besitzt
und Hal Chlor, Brom oder Jod bedeutet,

mit einem Aldehyd bzw. Keton der oben angegebenen allgemeinen Formel VIII, in der $R^4$ die oben angegebene Bedeutung von $R^{4'}$ besitzt, und mit Dimethylsulfid in wässrigem Medium umsetzt.

Diese Umsetzung wird zweckmässigerweise als Eintopfverfahren in Gegenwart eines inerten organischen Lösungsmittels und einer Base durchgeführt. Vorzugsweise wird deshalb in einem wässrig-organischen System gearbeitet. Als organische Lösungsmittel eignen sich insbesondere aliphatische und aromatische Kohlenwasserstoffe, wie n-Hexan, Benzol und Toluol; Alkohole, wie Methanol, Aethanol und Isopropanol; und aliphatische und cyclische Aether, wie Diäthyläther, Tetrahydrofuran und Dioxan. Als Basen sind wasserlösliche Alkalihydroxide, wie Natriumhydroxid und Kaliumhydroxid, bevorzugt. Es wird im allgemeinen zwischen der Raumtemperatur und der Rückflusstemperatur des Reaktionsgemisches gearbeitet.

Die Ausgangsmaterialien der allgemeinen Formel III können z.B. durch Umsetzung eines Epoxids der Formel II mit Chlor- oder Bromwasserstoffsäure oder mit einer Alkansulfonsäure bzw. einer aromatischen Sulfonsäure analog zur Verfahrensvariante a) hergestellt werden.

Diejenigen Ausgangsmaterialien der allgemeinen Formel V, in der $R^{1''}$ gegebenenfalls substituiertes $C_{3-6}$-Alkenyl, $C_{3-6}$-Alkinyl oder Aryl-$C_{1-3}$-alkyl, wie oben näher definiert, bedeutet, sind neu und können hergestellt werden, indem man ein Keton der allgemeinen Formel

$$R - CH_2 - \underset{\underset{O}{\|}}{C} - R^{4'} \qquad X$$

worin R und $R^{4'}$ die oben angegebenen Bedeutungen besitzen,

mit einem Alkylierungsmittel der allgemeinen Formel

$$R^{1''''} - U \qquad XI$$

worin $R^{1''''}$ gegebenenfalls mit 1 bis 3 Halogenatomen substituiertes $C_{3-6}$-Alkenyl oder $C_{3-6}$-Alkinyl; oder gegebenenfalls im Arylkern mono-, di- oder trisubstituiertes Aryl-$C_{1-3}$-alkyl, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind,

und U eine Abgangsgruppe, insbesondere Chlor, Brom oder Jod, bedeuten,

behandelt.

Bei der Umsetzung wird mit Vorteil das Keton der Formel X mit einer Base, wie Natriumhydrid oder Lithiumdiisopropylamid, in einem Verdünnungsmittel, vorzugsweise einem aprotischen organischen Lösungsmittel, wie Tetrahydrofuran, Dimethoxyäthan oder Dimethylformamid, bei Reaktionstemperaturen zwischen -70°C und 50°C in ein Anion übergeführt, welches dann mit dem Alkylierungsmittel der Formel XI versetzt wird. Die Behandlung des Ketons der Formel X mit dem Alkylierungsmittel der Formel XI kann auch in einem wässrig-organischen Zweiphasensystem in Gegenwart eines Phasentransferkatalysators, d.h. unter den Bedingungen der Phasentransferkatalyse (siehe z.B. J. Dockx, Synthesis (1973), 441), erfolgen.

Auch die übrigen Ausgangsmaterialien der allgemeinen Formel V, d.h. diejenigen Ketone der Formel V, in der $R^{1''}$ die oben angegebene Bedeutung von $R^{1'''}$ besitzt, sind neu. Sie können dadurch hergestellt werden, dass man einen Thioäther der allgemeinen Formel VII mit einem Ester der allgemeinen Formel

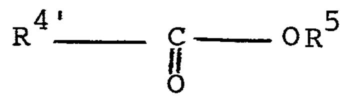 XII

worin $R^{4'}$ die oben angegebene Bedeutung besitzt
und $R^5$ $C_{1-4}$-Alkyl oder Phenyl bedeutet,

umsetzt. Die Umsetzung wird zweckmässigerweise unter den in Zusammenhang mit der Verfahrensvariante e) oben angegebenen Reaktionsbedingungen durchgeführt, wobei anstelle des Ketons der Formel VIII das Ester der Formel XII umgesetzt wird.

Die Ausgangsmaterialien der allgemeinen Formel V bilden einen weiteren Gegenstand der vorliegenden Erfindung.

Bei den Ausgangsmaterialien der allgemeinen Formel I' handelt es sich um diejenigen Verbindungen der Formel I, in der $R^2$ Hydroxy und $R^4$ 3-Pyridyl, 2-Pyrazinyl oder 5-Pyrimidinyl bedeuten. Diese Verbindungen können z.B. gemäss den Verfahrensvarianten a) bis c) und e) hergestellt werden.

Die Ausgangsmaterialien der allgemeinen Formel I" stellen ebenfalls eine Unterklasse der Verbindungen der allgemeinen Formel I dar und können z.B. gemäss den Verfahrensvarianten a) bis e) hergestellt werden.

Die Ausgangsmaterialien der allgemeinen Formeln IV, VI bis IX, XI und XII sind entweder bekannt oder können nach an sich bekannten Methoden hergestellt werden.

Die als Ausgangsmaterialien dienenden Ketone der allgemeinen Formel X sind entweder bekannt oder können nach an sich bekannten Methoden hergestellt werden (siehe z.B. die deutsche Offenlegungsschrift Nr. 2 221 546). Besonders bevorzugt ist jedoch das folgende Verfahren:

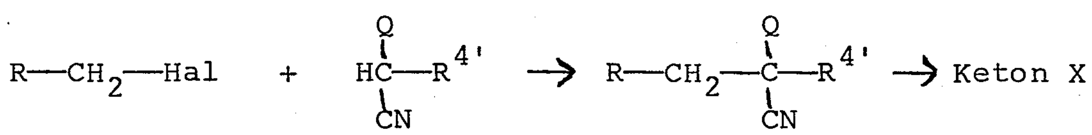


IX XIII XIV

worin R, $R^{4'}$ und Hal die oben angegebenen Bedeutungen besitzen

und Q einen disubstituierten Aminorest, z.B. Dimethylamino, Diäthylamino, Piperidino oder Morpholino, oder die Trimethylsilyloxygruppe bedeutet.

Die Umsetzung der Verbindung der Formel IX mit der Verbindung der Formel XIII kann z.B. unter den Bedingungen der Phasentransferkatalyse (siehe z.B. J. Dockx, Synthesis

(1973), 441) oder unter Verwendung einer Base, wie z.B. Natriumhydrid oder Lithiumdiisopropylamid, in einem inerten Lösungsmittel, wie z.B. einem aliphatischen oder cyclischen Aether, z.B. Tetrahydrofuran oder Dimethoxyäthan, in einem Temperaturbereich zwischen -70°C und 50°C, vorzugsweise zwischen -30°C und der Raumtemperatur, und unter Ausschluss von Wasser durchgeführt werden. Die Verbindung der Formel XIII, in der Q die Trimethylsilyloxygruppe bedeutet, wird vorzugsweise in Gegenwart einer Base umgesetzt. Das so erhaltene Produkt der Formel XIV lässt sich hierauf durch Hydrolyse, z.B. durch konventionelle Behandlung mit einer wässrigen Säure, in das Keton der Formel X überführen. Als Säuren kommen für diesen Zweck insbesondere starke anorganische Säuren, wie Schwefelsäure, Chlorwasserstoffsäure und Bromwasserstoffsäure, sowie Sulfonsäuren, wie Benzolsulfonsäure und p-Toluolsulfonsäure, in Frage. Die Hydrolyse wird zweckmässigerweise in einem Temperaturbereich zwischen 20°C und der Rückflusstemperatur des Reaktionsgemisches, vorzugsweise zwischen 80°C und 100°C, ausgeführt.

Die Ausgangsmaterialien der allgemeinen Formel XIII sind entweder bekannt oder können nach an sich bekannten Methoden hergestellt werden, z.B. aus den entsprechenden Aldehyden der Formel $R^{4'}$-CHO [siehe z.B. E. Leete et al., J. Org. Chem. 43, 2122 (1978) und W. Lidy et al., Chem. Ber. 106, 587 (1973)].

Die erfindungsgemässen Verbindungen, d.h. die Verbindungen der Formel I und deren Säureadditionssalze, besitzen fungizide Wirkung und können dementsprechend zur Bekämpfung von Fungi in der Landwirtschaft und im Gartenbau Verwendung finden. Sie eignen sich insbesondere zur Hemmung des Wachstums oder zur Vernichtung von phytopathogenen Pilzen auf Pflanzenteilen, z.B. Blättern, Stengeln, Wurzeln, Knollen, Früchten oder Blüten, und auf Saatgut sowie im Erdboden und sind besonders wirksam bei der Bekämpfung von Botrytis cinerea (Graufäule); von echten Mehltaupilzen, wie beispielsweise Uncinula necator (Rebenmehltau), Erysiphe cichoracearum (Gurkenmehltau), Podosphaera leucotricha (Apfelmehltau) und

Erysiphe graminis (Gerstenmehltau); von Venturia inaequalis (Apfelschorf); und von Schadpilzen der Gattungen Puccinia, Uromyces, Hemileia, Rhizoctonia, Penicillium, Septoria, Corticium, Cercospora, Helminthosporium und Alternaria.

Einzelne Vertreter der erfindungsgemässen Verbindungen besitzen zudem eine ausgeprägte Wirkung gegen holzzerstörende Pilze, wie z.B. Coniophora puteana und Gloeophyllum trabeum.

Die erfindungsgemässen Verbindungen zeichnen sich durch lokale und bzw. oder systemische Wirkung aus.

Die erfindungsgemässen Verbindungen wirken unter Gewächshausbedingungen bereits bei einer Konzentration von 1 mg bis 500 mg Wirksubstanz pro Liter Spritzbrühe. Im Freiland werden vorteilhaft Konzentrationen von 25 g bis 1000 g Wirkstoff der Formel I pro Hektar und Behandlung zur Anwendung gebracht. Zur Bekämpfung von samenbürtigen Pilzen im Beizverfahren werden mit Vorteil 0,05 g bis 1,5 g Wirkstoff der Formel I pro kg Samen verwendet.

Auch die oben erwähnten Ketone der allgemeinen Formel V sowie ihre N-Oxide sind als Fungizide wertvoll, da sie ein ähnliches Aktivitätsspektrum wie die Verbindungen der Formel I aufweisen. Diese Verbindungen können dementsprechend auch zur Bekämpfung von Fungi in der Landwirtschaft und im Gartenbau eingesetzt werden, und zwar auf gleiche Weise wie die Verbindungen der Formel I. Die N-Oxide von V können durch N-Oxydation der Ketone der Formel V hergestellt werden, und zwar analog zur Verfahrensvariante f). Auch diese N-Oxide bilden einen Gegenstand der vorliegenden Erfindung.

Die erfindungsgemässen Verbindungen können zu verschiedenartigen Mitteln, z.B. Lösungen, Suspensionen, Emulsionen, emulgierbaren Konzentraten und pulverförmigen Präparaten, formuliert werden. Die erfindungsgemässen fungiziden Mittel sind dadurch gekennzeichnet, dass sie eine wirksame Menge mindestens einer Verbindung der allgemeinen

Formel I, wie oben definiert, oder eines Säureadditionssalzes einer solchen Verbindung sowie Formulierungshilfsstoffe enthalten. Die Mittel enthalten zweckmässigerweise zumindest einen der folgenden Formulierungshilfsstoffe:

Feste Trägerstoffe; Lösungs- bzw. Dispersionsmittel; Tenside (Netz- und Emulgiermittel); Dispergatoren (ohne Tensidwirkung); und Stabilisatoren.

Als feste Trägerstoffe kommen im wesentlichen in Frage: natürliche Mineralstoffe, wie Kaolin, Tonerden, Kieselgur, Talkum, Bentonit, Kreide, z.B. Schlämmkreide, Magnesiumcarbonat, Kalkstein, Quarz, Dolomit, Attapulgit, Montmorillonit und Diatomeenerde; synthetische Mineralstoffe, wie hochdisperse Kieselsäure,Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Granulate oder Pulver vorliegen können.

Als Lösungs- bzw. Dispersionsmittel kommen im wesentlichen in Frage: Aromaten, wie Toluol, Xylole, Benzol und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungs- bzw. Dispersionsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Im

Falle der Benutzung von Wasser als Lösungsmittel können z.B. auch organische Lösungsmittel als Hilfslösungsmittel verwendet werden.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäure, Natriumsalze von Maleinsäureanhydrid-Diisobutylen-Copolymeren, Natrium- und Ammoniumsalze von sulfonierten Polykondensationsprodukte aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose,

Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butylhydroxytoluol; UV-Absorber, z.B. substituierte Benzophenone, Diphenylacrylnitrilsäureester und Zimtsäureester; und Deaktivatoren, z.B. Salze der Aethylendiamintetraessigsäure und Polyglykole.

Die erfindungsgemässen fungiziden Mittel können neben den Wirkstoffen der Formel I auch andere Wirkstoffe enthalten, z.B. anderweitige fungizide Mittel, insektizide und akarizide Mittel, Bakterizide, Pflanzenwachstumsregulatoren und Düngemittel. Solche Kombinationsmittel eignen sich zur Verbreiterung des Wirkungsspektrums oder zur spezifischen Beeinflussung des Pflanzenwachstums.

Im allgemeinen enthalten die erfindungsgemässen fungiziden Mittel, je nach deren Art, zwischen 0,0001 und 95 Gewichtsprozent an erfindungsgemässer Verbindung bzw. erfindungsgemässen Verbindungen als Wirkstoff(en). Sie können in einer Form vorliegen, die sich für die Lagerung und den Transport eignet. In solchen Formen, z.B. emulgierbaren Konzentraten, ist die Wirkstoffkonzentration normalerweise im höheren Bereich der obigen Konzentrationsreihe. Diese Formen können dann mit gleichen oder verschiedenen Formulierungshilfsstoffen bis zu Wirkstoffkonzentrationen verdünnt werden, die sich für den praktischen Gebrauch eignen, und solche Konzentrationen liegen normalerweise im niedrigeren Bereich der obigen Konzentrationsreihe. Emulgierbare Konzentrate enthalten im allgemeinen 5 bis 95 Gewichtsprozent, vorzugsweise 25 bis 75 Gewichtsprozent, der Verbindung bzw. Verbindungen der Formel I. Als Anwendungsformen kommen u.a. gebrauchsfertige Lösungen, Emulsionen und Suspensionen, die sich beispielsweise als Spritzbrühen eignen, in Frage. In solchen Spritzbrühen können z.B. Konzentrationen zwischen

0,0001 und 20 Gewichtsprozent vorliegen. Im Ultra-Low-Volume-Verfahren können Spritzbrühen formuliert werden, in denen die Wirkstoffkonzentration vorzugsweise von 0,5 bis 20 Gewichtsprozent beträgt, während die im Low-Volume-Verfahren und im High-Volume-Verfahren formulierten Spritzbrühen vorzugsweise eine Wirkstoffkonzentration von 0,02 bis 1,0 bzw. 0,002 bis 0,1 Gewichtsprozent aufweisen.

Die erfindungsgemässen fungiziden Mittel können dadurch hergestellt werden, dass man mindestens eine Verbindung der allgemeinen Formel I bzw. ein Säureadditionssalz einer solchen Verbindung mit Formulierungshilfsstoffen vermischt.

Die Herstellung der Mittel kann in bekannter Weise durchgeführt werden, z.B. durch Vermischen des Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln oder von Dispergatoren, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem festen Trägerstoff vermischt werden, z.B. durch Zusammenmahlen; oder man kann den festen Trägerstoff mit einer Lösung oder Suspension des Wirkstoffs imprägnieren und dann das Lösungs- bzw. Dispersionsmittel durch Abdunsten, Erhitzen oder durch Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die erfindungsgemässen Verbindungen können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen

Produktes vermischt werden.

Wenn gewünscht, kann eine erfindungsgemässe Verbindung in einem mit Wasser nicht mischbaren Lösungsmittel, wie beispielsweise einem alicyclischen Keton, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Mittel kann nach den im Pflanzenschutz bzw. in der Landwirtschaft üblichen Applikationsmethoden erfolgen. Das erfindungsgemässe Verfahren zur Bekämpfung von Fungi ist dadurch gekennzeichnet, dass man das zu schützende Gut, z.B. Pflanzen, Pflanzenteile bzw. Samen, mit einer wirksamen Menge einer erfindungsgemässen Verbindung bzw. eines erfindungsgemässen Mittels behandelt.

Die nachstehenden Beispiele illustrieren die Erfindung.

I. Herstellung der Wirkstoff der Formel I:

Beispiel 1

3 g 2-(2,4-Dichlorphenyl)-1-(3-pyridyl)-4-penten-1-on werden in 20 ml Methanol gelöst und mit 0,5 g Natriumborhydrid versetzt. Nach einstündiger Reaktionszeit bei Raumtemperatur wird das Reaktionsgemisch etwas eingeengt, auf Eiswasser gegossen und mit Diäthyläther extrahiert. Die organische Phase wird gewaschen, über wasserfreiem Natriumsulfat getrocknet und das Lösungsmittel unter vermindertem Druck entfernt. Man erhält α-(α-Allyl-2,4-dichlorbenzyl)-3-pyridylmethanol als schwach bräunliches Oel.

Auf analoge Weise erhält man:

aus 2-(2,4-Dichlorphenyl)-1,3-di-(3-pyridyl)-1-propanon durch Reduktion mit Natriumborhydrid das 2-(2,4-Dichlorphenyl)-1,3-di-(3-pyridyl)-1-propanol, Smp. 65-67°C;

aus 2-(2,4-Dichlorphenyl)-4-methyl-1-(3-pyridyl)-4-penten-1-on durch Reduktion mit Natriumborhydrid das α-[2,4-Dichlor-α-(2-methallyl)-benzyl]-3-pyridylmethanol als gelbliches Oel;

aus 2-(2,4-Dichlorphenyl)-1-(3-pyridyl)-4-pentin-1-on durch Reduktion mit Natriumborhydrid das α-[2,4-Dichlor-α-(2-propinyl)-benzyl]-3-pyridylmethanol als gelbliches Oel;

aus 2-(2,4-Dichlorphenyl)-3-phenyl-1-(3-pyridyl)-1-propanon durch Reduktion mit Natriumborhydrid das α-[α-(2,4-Dichlorphenyl)-phenäthyl]-3-pyridylmethanol, Smp. 120-122°C;

aus 2-(2,4-Dichlorphenyl)-1-(2-pyrazinyl)-4-penten-1-on durch Reduktion mit Natriumborhydrid das α-(α-Allyl-2,4-dichlorbenzyl)-2-pyrazinylmethanol als gelbliches Oel;

aus 2-(2,4-Dichlorphenyl)-1-(5-pyrimidinyl)-4-pentin-1-on durch Reduktion mit Natriumborhydrid das α-[2,4-Dichlor-α-(2-propinyl)-benzyl]-5-pyrimidinylmethanol, Smp. 138-140°C;

aus 2-(2,4-Dichlorphenyl)-2-phenylthio-1-(3-pyridyl)-äthanon durch Reduktion mit Natriumborhydrid das α-[2,4-Dichlor-α-phenylthio-benzyl]-3-pyridylmethanol, Smp. 119-121°C;

aus 4,5-Dibrom-2-(2,4-dichlorphenyl)-1-(3-pyridyl)-4-penten-1-on durch Reduktion mit Natriumborhydrid das α-[α-(2,3-Dibromallyl)-2,4-dichlorbenzyl]-3-pyridylmethanol, Smp. 108-114°C;

aus 2-(2,4-Dichlorphenyl)-5-methyl-1-(3-pyridyl)-4-hexen-1-on durch Reduktion mit Natriumborhydrid das α-[2,4-Dichlor-α-(3-methyl-2-butenyl)-benzyl]-3-pyridylmethanol, Smp. 97-100°C;

aus 2-(2,4-Dichlorphenyl)-3-(p-nitrophenyl)-1-(3-pyridyl)-1-propanon durch Reduktion mit Natriumborhydrid das α-[α-(2,4-Dichlorphenyl)-p-nitrophenäthyl]-3-pyridylmethanol, Smp. 164-165°C;

aus 2-(2,4-Dichlorphenyl)-3-(2-thienyl)-1-(3-pyridyl)-1-propanon durch Reduktion mit Natriumborhydrid das 2-(2,4-Dichlorphenyl)-3-(2-thienyl)-1-(3-pyridyl)-1-propanol, Smp. 112-114°C;

aus 2-(2,4-Dichlorphenyl)-3-(2-furyl)-1-(3-pyridyl)-1-propanon durch Reduktion mit Natriumborhydrid das α-[2,4-Dichlor-α-(2-furfuryl)-benzyl]-3-pyridylmethanol, Smp. 132-133°C;

und aus einem 4:1-Gemisch von 4- und 5-Chlor-2-(2,4-dichlorphenyl)-1-(3-pyridyl)-4-penten-1-on durch Reduktion mit Natriumborhydrid ein 4:1-Gemisch von α-[1-(2,4-Dichlorphenyl)-3- und 4-chlor-3-butenyl]-3-pyridylmethanol als gelbliche

amorphe Substanz.

Beispiel 2

Zu einer Lösung von Lithiumdiisopropylamid (aus 3,2 g Diisopropylamin und einer äquivalenten Menge n-Butyllithium) in 7,1 g Hexamethylphosphorsäuretriamid und 350 ml Tetrahydrofuran bei -20°C tropft man langsam 8 g 2,4-Dichlor-α-(4-chlorphenylthio)-toluol, gelöst in wenig Tetrahydrofuran, zu und lässt 1 Stunde bei dieser Temperatur rühren. Dann tropft man 2,54 g 3-Pyridincarbaldehyd, verdünnt mit wenig Tetrahydrofuran, zu und lässt die Temperatur des Reaktionsgemisches langsam auf Raumtemperatur ansteigen. Es wird mit wenig Wasser versetzt und etwas eingeengt. Anschliessend giesst man das Reaktionsgemisch auf Eiswasser, extrahiert mit Aethylacetat, trocknet die organische Phase über wasserfreiem Natriumsulfat und entfernt das Lösungsmittel. Das Rohprodukt wird an Kieselgel mit n-Hexan/Aethylacetat (1:1) chromatographisch gereinigt. Man erhält hellgelbe Kristalle von α-[2,4-Dichlor-α-(4-chlorphenylthio)-benzyl]-3-pyridylmethanol, Smp. 140-141°C.

Auf analoge Weise erhält man aus 2,4-Dichlor-α-(2-pyridylthio)-toluol und 3-Pyridincarbaldehyd bräunliche Kristalle von α-[2,4-Dichlor-α-(2-pyridylthio)-benzyl]-3-pyridylmethanol, Smp. 121-123°C.

Beispiel 3

Zu einer Lösung von Methylmagnesiumjodid (hergestellt aus 0,62 g Magnesiumspänen und 3,8 g Methyljodid) in 70 ml Diäthyläther werden 6,0 g 2-(2,4-Dichlorphenyl)-1-(3-pyridyl)-4-penten-1-on, verdünnt mit 20 ml Diäthyläther, getropft. Nach einer Reaktionszeit von 45 Minuten bei Raumtemperatur wird das Reaktionsgemisch mit gesättigter wässriger Ammoniumchloridlösung versetzt, mit Wasser verdünnt und anschliessend mit Diäthyläther extrahiert. Nach Trocknen der organischen Phase über wasserfreiem Natriumsulfat wird

das Lösungsmittel unter vermindertem Druck entfernt. Man erhält das α-(α-Allyl-2,4-dichlorbenzyl)-α-methyl-3-pyridylmethanol als rotbraune amorphe Substanz.

Auf analoge Weise erhält man:

aus 2-(2,4-Dichlorphenyl)-1-(3-pyridyl)-4-penten-1-on und Aethylmagnesiumbromid das α-(α-Allyl-2,4-dichlorbenzyl)-α-äthyl-3-pyridylmethanol, Smp. 105-110°C;

aus 2-(2,4-Dichlorphenyl)-3-(2-furyl)-1-(3-pyridyl)-1-propanon und Methylmagnesiumjodid das α-[2,4-Dichlor-α-(2-furfuryl)-benzyl]-α-methyl-3-pyridylmethanol als amorphe rötliche Substanz;

aus 2-(2,4-Dichlorphenyl)-3-(2-thienyl)-1-(3-pyridyl)-1-propanon und Methylmagnesiumjodid das 3-(2,4-Dichlorphenyl)-4-(2-thienyl)-2-(3-pyridyl)-2-butanol als amorphe orange Substanz;

und aus einem 4:1-Gemisch von 4- und 5-Chlor-2-(2,4-dichlorphenyl)-1-(3-pyridyl)-4-penten-1-on mit Methylmagnesiumjodid ein 4:1-Gemisch von α-[1-(2,4-Dichlorphenyl)-3- und 4-chlor-3-butenyl]-α-methyl-3-pyridylmethanol als amorphe gelbliche Substanz mit Schmelzbereich von 123-152°C.

## Beispiel 4

4 g α-(α-Allyl-2,4-dichlorbenzyl)-3-pyridylmethanol werden mit 20 ml Phosphoroxychlorid während 1 Stunde bei 90°C gerührt. Dann wird das Reaktionsgemisch auf Eiswasser gegossen, mit Sodalösung besisch gestellt und mit Diäthyläther extrahiert. Die organische Phase wird über wasserfreiem Natriumsulfat getrocknet, das Lösungsmittel unter vermindertem Drück entfernt und das erhaltene Produkt an Kieselgel mit n-Hexan/Aethylacetat (3:1) chromatographisch gereinigt. Man erhält das 3-[1-Chlor-2-(2,4-dichlorphenyl)-4-pentenyl]-pyridin als gelbliches Oel.

## Beispiel 5

1,5 g 3-(2,4-Dichlorphenyl)-4-(2-thienyl)-2-(3-pyridyl)-2-butanol, gelöst in 20 ml Chloroform, werden mit 1,5 g 3-Chlorperbenzoesäure versetzt und während 18 Stunden bei 4°C aufbewahrt. Das Reaktionsgemisch wird mit Wasser und Chloroform versetzt, die organische Phase mit wässriger Kaliumcarbonatlösung gewaschen, anschliessend über wasserfreiem Natriumsulfat getrocknet und unter vermindertem Druck eingeengt. Man erhält das α-[2,4-Dichlor-α-(2-thenyl)-benzyl]-α-methyl-3-pyridylmethanol-1-oxid, das aus n-Hexan/Aethylacetat umkristallisiert. Smp. 95°C (unter Zersetzung).

## II. Herstellung der Ausgangsmaterialien:

## Beispiel 6

Eine Lösung von 25 g 2,4-Dichlorbenzyl-(3-pyridyl)-keton in 120 ml Dimethylformamid wird bei 0°C portionenweise mit 4 g Natriumhydrid (60%ige Dispersion in Oel) versetzt. Nach 2 Stunden bei Raumtemperatur werden 13,6 g Allylbromid zugefügt, und es wird weitere 3 Stunden bei Raumtemperatur gerührt. Dann wird auf Wasser gegossen, mit Diäthyläther extrahiert, die organische Phase gewaschen und über wasserfreiem Natriumsulfat getrocknet und anschliessend eingeengt. Durch Chromatographie an Kieselgel mit Aethylacetat erhält man 2-(2,4-Dichlorphenyl)-1-(3-pyridyl)-4-penten-1-on als Oel.

Auf analoge Weise erhält man:

aus 2,4-Dichlorbenzyl-(3-pyridyl)-keton und 3-Picolylchlorid das 2-(2,4-Dichlorphenyl)-1,3-di-(3-pyridyl)-1-propanon als Oel;

aus 2,4-Dichlorbenzyl-(3-pyridyl)-keton und β-Methallylchlorid das 2-(2,4-Dichlorphenyl)-4-methyl-1-(3-pyridyl)-4-penten-1-on als bräunliches Oel;

aus 2,4-Dichlorbenzyl-(3-pyridyl)-keton und Propargylbromid das 2-(2,4-Dichlorphenyl)-1-(3-pyridyl)-4-pentin-1-on als bräunliches Oel;

aus 2,4-Dichlorbenzyl-(3-pyridyl)-keton und Benzylbromid das 2-(2,4-Dichlorphenyl)-3-phenyl-1-(3-pyridyl)-1-propanon als bräunliches Oel;

aus 2,4-Dichlorbenzyl-(3-pyridyl)-keton und 2-Brommethylthiophen das 2-(2,4-Dichlorphenyl)-3-(2-thienyl)-1-(3-pyridyl)-1-propanon als rötliches Oel;

aus 2,4-Dichlorbenzyl-(3-pyridyl)-keton und p-Nitrobenzylchlorid das 2-(2,4-Dichlorphenyl)-3-(p-nitrophenyl)-1-(3-pyridyl)-1-propanon als rötliches Oel;

aus 2,4-Dichlorbenzyl-(3-pyridyl)-keton und 1-Brom-3-methyl-2-buten das 2-(2,4-Dichlorphenyl)-5-methyl-1-(3-pyridyl)-4-hexen-1-on als orangefarbenes Oel;

aus 2,4-Dichlorbenzyl-(3-pyridyl)-keton und 1,2,3-Tribrom-propen das 4,5-Dibrom-2-(2,4-dichlorphenyl)-1-(3-pyridyl)-4-penten-1-on Smp. 76-83°C;

aus 2,4-Dichlorbenzyl-(3-pyridyl)-keton und 3-Brom-2-chlor-1-propen, das mit ca. 20% 3-Brom-1-chlor-1-propen verunreinigt war, ein 4:1-Gemisch von 4- und 5-Chlor-2-(2,4-dichlorphenyl)-1-(3-pyridyl)-4-penten-1-on als orangefarbenes Oel;

aus 2,4-Dichlorbenzyl-(3-pyridyl)-keton und 2-Furfurylchlorid das 2-(2,4-Dichlorphenyl)-3-(2-furyl)-1-(3-pyridyl)-1-propanon als Oel;

aus 2,4-Dichlorbenzyl-(2-pyrazinyl)-keton und Allylbromid das 2-(2,4-Dichlorphenyl)-1-(2-pyrazinyl)-4-penten-1-on als bräunliches Oel;

und aus 2,4-Dichlorbenzyl-(5-pyrimidinyl)-keton und Propargylbromid das 2-(2,4-Dichlorphenyl)-1-(5-pyrimidinyl)-4-pentin-1-on als Oel.

Die dabei als Ausgangsmaterialien dienenden neuen Ketone können wie folgt hergestellt werden:

Zu einer Lösung von Lithiumdiisopropylamid (aus 5,4 g Diisopropylamin und einer äquivalenten Menge n-Butyllithium) in 50 ml Tetrahydrofuran bei -70° wird eine Lösung von 10 g α-(Trimethylsilyloxy)-α-(2-pyrazinyl)-acetonitril (aus 2-Pyrazincarbaldehyd und Trimethylsilylcyanid) in 30 ml Tetrahydrofuran zugetropft. Nach 1 Stunde gibt man bei gleicher Temperatur 9,4 g 2,4-Dichlorbenzylchlorid, verdünnt mit 10 ml Tetrahydrofuran, zu und lässt langsam auf Raumtemperatur erwärmen. Anschliessend fügt man 125 ml 2n-Salzsäure zu und lässt 10 Stunden bei Raumtemperatur reagieren. Dann wird basisch gestellt, mit Aethylacetat extrahiert, eingeengt und das Rohprodukt an Kieselgel mit n-Hexan/Aethylacetat (2:1) chromatographisch gereinigt. Man erhält 2,4-Dichlorbenzyl-(2-pyrazinyl)-keton, Smp. 109-111°C.

Auf analoge Weise erhält man aus α-(Trimethylsilyloxy)-α-(5-pyrimidinyl)-acetonitril und 2,4-Dichlorbenzylchlorid das 2,4-Dichlorbenzyl-(5-pyrimidinyl)-keton, Smp. 135-140°C.

## Beispiel 7

Zu einer Lösung von Lithiumdiisopropylamid (aus 7,67 g Diisopropylamin und einer äquivalenten Menge n-Butyllithium) in 61,9 g Hexamethylphosphorsäuretriamid und 400 ml Tetrahydrofuran bei -10°C tropft man 17 g 2,4-Dichlor-α-phenylthio-toluol, gelöst in wenig Tetrahydrofuran, zu. Man lässt 1 Stunde rühren, fügt 4,33 g Nicotinsäuremethylester zu und lässt dann 1 Stunde bei Raumtemperatur rühren.

Nach Zufügen von wenig Wasser wird unter vermindertem Druck eingeengt, auf Eiswasser gegossen und mit Aethylacetat extrahiert. Die organische Phase wird über wasserfreiem Natriumsulfat getrocknet und eingeengt. Das Rohprodukt wird an Kieselgel mit n-Hexan/Aethylacetat (1:1) chromatographisch gereinigt. Man erhält 2-(2,4-Dichlorphenyl)-2-phenylthio-1-(3-pyridyl)-äthanon als bräunliches Oel.

III. Formulierungsbeispiele:

## Beispiel 8

1. Spritzpulver (für flüssige oder unter 75°C schmelzende Wirkstoffe)

| | Gewichtsteile |
|---|---|
| Wirkstoff der Formel I bzw. V | 50 |
| Hydratisierte Kieselsäure | 37 |
| Kaolin | 5 |
| Alkylphenoläthoxylat | 4 |
| Natrium-polynaphthalinsulfonat | 4 |
| | 100 |

Der flüssige oder geschmolzene Wirkstoff wird auf die Kieselsäure aufgezogen, die übrigen Komponenten zugemischt und das Ganze in einer geeigneten Mühle feingemahlen.

2. Spritzpulver (für feste, über 75°C schmelzende Wirkstoffe)

| | Gewichtsteile |
|---|---|
| Wirkstoff der Formel I bzw. V | 50 |
| Hydratisierte Kieselsäure | 5 |
| Kaolin | 42 |
| Natrium-laurylsulfat | 1 |
| Natrium-lignosulfonat | 2 |
| | 100 |

Die Komponenten werden miteinander vermischt, und das Ganze wird dann in einer geeigneten Mühle feingemahlen.

Beispiel 9

Emulgierbares Konzentrat (für bei 20-25°C flüssige Wirkstoffe)

| | | Gewichtsteile |
|---|---|---|
| Wirkstoff der Formel I bzw. V | | 500 |
| Ricinusöl-äthoxylat | | 100 |
| Calcium-dodecylbenzolsulfonat | | 25 |
| Gemisch von $C_{10}$-Alkylbenzolen | ad | 1000 Vol.-Teile |

Die Komponenten werden miteinander vermischt, bis eine klare Lösung entsteht.

Patentansprüche

1. Verbindungen der allgemeinen Formel

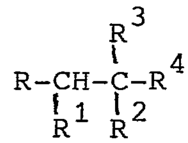

I

worin R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ gegebenenfalls mit 1 bis 3 Halogenatomen substituiertes $C_{3-6}$-Alkenyl, $C_{3-6}$-Alkinyl, $C_{3-6}$-Alkenyloxy, $C_{3-6}$-Alkinyloxy, $C_{3-6}$-Alkenylthio oder $C_{3-6}$-Alkinylthio; oder gegebenenfalls im Arylkern mono-, di- oder trisubstituiertes Aryl-$C_{1-3}$-alkyl, Aryloxy oder Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind,

$R^2$ Hydroxy, Chlor oder Brom,

$R^3$ Wasserstoff oder $C_{1-4}$-Alkyl

und $R^4$ 3-Pyridyl, 3-Pyridyl-1-oxid, 2-Pyrazinyl, 2-Pyrazinyl-1-oxid, 2-Pyrazinyl-4-oxid, 2-Pyrazinyl-1,4-dioxid, 5-Pyrimidinyl, 5-Pyrimidinyl-1-oxid oder 5-Pyrimidinyl-1,3-dioxid bedeuten,

und deren Säureadditionssalze.

2. Verbindungen nach Anspruch 1, worin R 2,4-Dichlorphenyl bedeutet.

3. Verbindungen nach Anspruch 1 oder 2, worin $R^1$ gegebenenfalls mit 1 bis 3 Halogenatomen substituiertes

$C_{3-6}$-Alkenyl oder $C_{3-6}$-Alkinyl, oder gegebenenfalls mono-, di- oder trisubstituiertes Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind.

4. Verbindungen nach einem der Ansprüche 1 bis 3, worin $R^3$ Wasserstoff oder Methyl bedeutet.

5. Verbindungen nach einem der Ansprüche 1 bis 4, worin $R^4$ 3-Pyridyl, 2-Pyrazinyl oder 5-Pyrimidinyl bedeutet.

6. α-(α-Allyl-2,4-dichlorbenzyl)-3-pyridylmethanol.

7. α-[2,4-Dichlor-α-(2-propinyl)-benzyl]-3-pyridylmethanol.

8. α-[α-(2,4-Dichlorphenyl)-phenäthyl]-3-pyridylmethanol.

9. α-(2,4-Dichlor-α-phenylthio-benzyl)-3-pyridylmethanol.

10. Eine Verbindung nach Anspruch 1, ausgewählt aus:

2-(2,4-Dichlorphenyl)-1,3-di-(3-pyridyl)-1-propanol,
α-[2,4-Dichlor-α-(2-methallyl)-benzyl]-3-pyridylmethanol,
α-(α-Allyl-2,4-dichlorbenzyl)-2-pyrazinylmethanol,
α-[2,4-Dichlor-α-(2-propinyl)-benzyl]-5-pyrimidinylmethanol,
α-[2,4-Dichlor-α-(4-chlorphenylthio)-benzyl]-3-pyridylmethanol,
α-[2,4-Dichlor-α-(2-pyridylthio)-benzyl]-3-pyridylmethanol,
α-[α-(2,3-Dibromallyl)-2,4-dichlorbenzyl]-3-pyridylmethanol,
2-(2,4-Dichlorphenyl)-3-(2-thienyl)-1-(3-pyridyl)-1-propanol,

α-[2,4-Dichlor-α-(2-furfuryl)-benzyl]-3-pyridylmethanol und

3-[1-Chlor-2-(2,4-dichlorphenyl)-4-pentenyl]-pyridin.

11. Eine Verbindung nach Anspruch 1, ausgewählt aus:

α-[2,4-Dichlor-α-(3-methyl-2-butenyl)-benzyl]-3-pyridylmethanol,

α-[α-(2,4-Dichlorphenyl)-p-nitrophenäthyl]-3-pyridylmethanol,

α-[1-(2,4-Dichlorphenyl)-3-chlor-3-butenyl]-3-pyridylmethanol,

α-[1-(2,4-Dichlorphenyl)-4-chlor-3-butenyl]-3-pyridylmethanol,

α-(α-Allyl-2,4-dichlorbenzyl)-α-methyl-3-pyridylmethanol,

α-(α-Allyl-2,4-dichlorbenzyl)-α-äthyl-3-pyridylmethanol,

α-[2,4-Dichlor-α-(2-furfuryl)-benzyl]-α-methyl-3-pyridylmethanol,

3-(2,4-Dichlorphenyl)-4-(2-thienyl)-2-(3-pyridyl)-2-butanol,

α-[1-(2,4-Dichlorphenyl)-3-chlor-3-butenyl]-α-methyl-3-pyridylmethanol,

α-[1-(2,4-Dichlorphenyl)-4-chlor-3-butenyl]-α-methyl-3-pyridylmethanol und

α-[2,4-Dichlor-α-(2-thenyl)-benzyl]-α-methyl-3-pyridylmethanol-1-oxid.

12. Verbindungen nach Anspruch 1 als fungizide Wirkstoffe.

13. Verbindungen der allgemeinen Formel

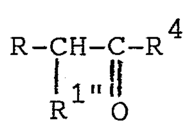

worin R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^{1''}$ gegebenenfalls mit 1 bis 3 Halogenatomen substituiertes $C_{3-6}$-Alkenyl, $C_{3-6}$-Alkinyl, $C_{3-6}$-Alkenylthio oder $C_{3-6}$-Alkinylthio; oder gegebenenfalls im Arylkern mono-, di- oder trisubstituiertes Aryl-$C_{1-3}$-alkyl oder Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind,

und $R^4$ 3-Pyridyl, 3-Pyridyl-1-oxid, 2-Pyrazinyl, 2-Pyrazinyl-1-oxid, 2-Pyrazinyl-4-oxid, 2-Pyrazinyl-1,4-dioxid, 5-Pyrimidinyl, 5-Pyrimidinyl-1-oxid oder 5-Pyrimidinyl-1,3-dioxid bedeuten.

14. Fungizides Mittel, dadurch gekennzeichnet, dass es eine wirksame Menge mindestens einer Verbindung der allgemeinen Formel

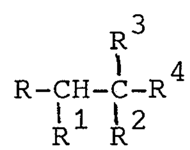

I

worin R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ gegebenenfalls mit 1 bis 3 Halogenatomen substituiertes $C_{3-6}$-Alkenyl, $C_{3-6}$-Alkinyl, $C_{3-6}$-Alkenyloxy, $C_{3-6}$-Alkinyloxy, $C_{3-6}$-Alkenylthio oder $C_{3-6}$-Alkinylthio; oder

gegebenenfalls im Arylkern mono-, di- oder trisubstituiertes Aryl-$C_{1-3}$-alkyl, Aryloxy oder Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind,

$R^2$ Hydroxy, Chlor oder Brom,

$R^3$ Wasserstoff oder $C_{1-4}$-Alkyl

und $R^4$ 3-Pyridyl, 3-Pyridyl-1-oxid, 2-Pyrazinyl, 2-Pyrazinyl-1-oxid, 2-Pyrazinyl-4-oxid, 2-Pyrazinyl-1,4-dioxid, 5-Pyrimidinyl, 5-Pyrimidinyl-1-oxid oder 5-Pyrimidinyl-1,3-dioxid bedeuten,

oder eines Säureadditionssalzes einer solchen Verbindung, sowie Formulierungshilfsstoffe enthält.

15. Fungizides Mittel nach Anspruch 14, dadurch gekennzeichnet, dass es eine wirksame Menge von α-(α-Allyl-2,4-dichlorbenzyl)-3-pyridylmethanol oder α-[2,4-Dichlor-α-(2-propinyl)-benzyl]-3-pyridylmethanol oder α-[α-(2,4-Dichlorphenyl)-phenäthyl]-3-pyridylmethanol oder α-(2,4-Dichlor-α-phenylthio-benzyl)-3-pyridylmethanol sowie Formulierungshilfsstoffe enthält.

16. Verfahren zur Herstellung von Verbindungen der allgemeinen Formel

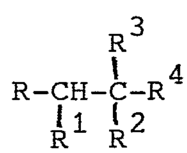

I

worin R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ gegebenenfalls mit 1 bis 3 Halogenatomen

substituiertes $C_{3-6}$-Alkenyl, $C_{3-6}$-Alkinyl, $C_{3-6}$-Alkenyloxy, $C_{3-6}$-Alkinyloxy, $C_{3-6}$-Alkenylthio oder $C_{3-6}$-Alkinylthio; oder gegebenenfalls im Arylkern mono-, di- oder trisubstituiertes Aryl-$C_{1-3}$-alkyl, Aryloxy oder Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind,

$R^2$ Hydroxy, Chlor oder Brom,

$R^3$ Wasserstoff oder $C_{1-4}$-Alkyl

und $R^4$ 3-Pyridyl, 3-Pyridyl-1-oxid, 2-Pyrazinyl, 2-Pyrazinyl-1-oxid, 2-Pyrazinyl-4-oxid, 2-Pyrazinyl-1,4-dioxid, 5-Pyrimidinyl, 5-Pyrimidinyl-1-oxid oder 5-Pyrimidinyl-1,3-dioxid bedeuten,

und von ihren Säureadditionssalzen, dadurch gekennzeichnet, dass man

a) zwecks Herstellung der Verbindungen der Formel I, worin $R^1$ gegebenenfalls substituiertes $C_{3-6}$-Alkenyloxy, $C_{3-6}$-Alkinyloxy oder Aryloxy, wie oben näher definiert, $R^2$ Hydroxy und $R^4$ 3-Pyridyl, 2-Pyrazinyl oder 5-Pyrimidinyl bedeuten, ein Epoxid der allgemeinen Formel

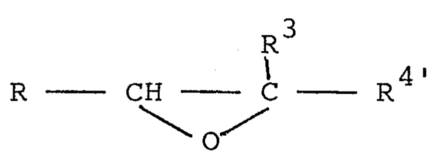

II

worin R und $R^3$ die oben angegebenen Bedeutungen besitzen

und $R^{4'}$ 3-Pyridyl, 2-Pyrazinyl oder 5-Pyrimidinyl bedeutet,

oder einen Alkohol der allgemeinen Formel

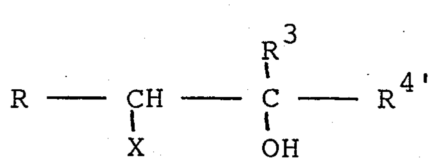

III

worin R, $R^3$ und $R^{4'}$ die oben angegebenen Bedeutungen besitzen

und X ein Halogenatom, einen Alkylsulfonatrest oder einen Arylsulfonatrest bedeutet,

mit einer Verbindung der allgemeinen Formel

$$R^{1'} — H \qquad IV$$

worin $R^{1'}$ gegebenenfalls mit 1 bis 3 Halogenatomen substituiertes $C_{3-6}$-Alkenyloxy oder $C_{3-6}$-Alkinyloxy; oder gegebenenfalls mono-, di- oder trisubstituiertes Aryloxy, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind, bedeutet,

umsetzt,

b) zwecks Herstellung der Verbindungen der Formel I, worin $R^1$ gegebenenfalls substituiertes $C_{3-6}$-Alkenyl, $C_{3-6}$-Alkinyl, $C_{3-6}$-Alkenylthio, $C_{3-6}$-Alkinylthio, Aryl-$C_{1-3}$-alkyl oder Arylthio, wie oben näher definiert, $R^2$ Hydroxy, $R^3$ Wasserstoff und $R^4$ 3-Pyridyl, 2-Pyrazinyl oder 5-Pyrimidinyl bedeuten, ein Keton der allgemeinen Formel

$$R — \underset{R^{1''}}{\underset{|}{CH}} — \underset{O}{\underset{\|}{C}} — R^{4'} \qquad V$$

worin R und $R^{4'}$ die oben angegebenen Bedeutungen besitzen

und $R^{1''}$ gegebenenfalls mit 1 bis 3 Halogenatomen substituiertes $C_{3-6}$-Alkenyl, $C_{3-6}$-Alkinyl, $C_{3-6}$-Alkenylthio oder $C_{3-6}$-Alkinylthio; oder gegebenenfalls im Arylkern mono-, di- oder trisubstituiertes Aryl-$C_{1-3}$-alkyl oder Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind, bedeutet,

reduziert,

c) zwecks Herstellung der Verbindungen der Formel I, worin $R^1$ gegebenenfalls substituiertes $C_{3-6}$-Alkenyl, $C_{3-6}$-Alkinyl, $C_{3-6}$-Alkenylthio, $C_{3-6}$-Alkinylthio, Aryl-$C_{1-3}$-alkyl oder Arylthio, wie oben näher definiert, $R^2$ Hydroxy, $R^3$ $C_{1-4}$-Alkyl und $R^4$ 3-Pyridyl, 2-Pyrazinyl oder 5-Pyrimidinyl bedeuten, ein Keton der allgemeinen Formel V, wie oben definiert, mit einer Verbindung der allgemeinen Formel

$$R^{3'} — Y \qquad VI$$

worin $R^{3'}$ $C_{1-4}$-Alkyl,
Y Lithium oder MgZ
und Z Halogen bedeuten,

umsetzt,

d) zwecks Herstellung der Verbindungen der Formel I, worin $R^2$ Chlor oder Brom und $R^4$ 3-Pyridyl, 2-Pyrazinyl oder 5-Pyrimidinyl bedeuten, einen Alkohol der allgemeinen Formel

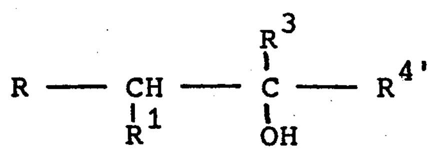

I'

worin R, $R^1$, $R^3$ und $R^{4'}$ die oben angegebenen Bedeutungen besitzen,

mit einem Chlorierungsmittel bzw. Bromierungsmittel behandelt,

e) zwecks Herstellung der Verbindungen der Formel I, worin $R^1$ gegebenenfalls substituiertes $C_{3-6}$-Alkenylthio, $C_{3-6}$-Alkinylthio oder Arylthio, wie oben näher definiert, und $R^2$ Hydroxy bedeuten, einen Thioäther der allgemeinen Formel

0117485

$R — CH_2 — R^{1'''}$ VII

worin R die oben angegebene Bedeutung besitzt und $R^{1'''}$ gegebenenfalls mit 1 bis 3 Halogenatomen substituiertes $C_{3-6}$-Alkenylthio oder $C_{3-6}$-Alkinylthio; oder gegebenenfalls mono-, di- oder trisubstituiertes Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind, bedeutet,

mit einem Aldehyd bzw. Keton der allgemeinen Formel

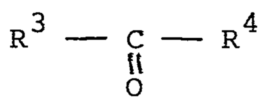

VIII

worin $R^3$ und $R^4$ die oben angegebenen Bedeutungen besitzen,

umsetzt, oder

f) zwecks Herstellung der Verbindungen der Formel I, worin $R^4$ 3-Pyridyl-1-oxid, 2-Pyrazinyl-1-oxid, 2-Pyrazinyl-4-oxid, 2-Pyrazinyl-1,4-dioxid, 5-Pyrimidinyl-1-oxid oder 5-Pyrimidinyl-1,3-dioxid bedeutet, ein Pyridin-, Pyrazin- bzw. Pyrimidinderivat der allgemeinen Formel

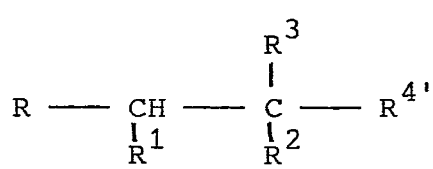

I"

worin R, $R^1$, $R^2$, $R^3$ und $R^{4'}$ die oben angegebenen Bedeutungen besitzen,

N-oxydiert

und erwünschtenfalls eine erhaltene Verbindung der Formel I mit einer Säure zur Herstellung des entsprechenden Säureadditionssalzes umsetzt.

17. Verfahren zur Bekämpfung von Fungi in der Landwirtschaft und im Gartenbau, dadurch gekennzeichnet, dass man das zu schützende Gut mit einer wirksamen Menge einer der in den Ansprüchen 1 bis 11 genannten Verbindungen bzw. eines in Anspruch 14 oder 15 genannten Mittels behandelt.

18. Verwendung einer der in den Ansprüchen 1 bis 11 genannten Verbindungen bzw. eines in Anspruch 14 oder 15 genannten Mittels zur Bekämpfung von Fungi in der Landwirtschaft und im Gartenbau.

***

0117485
DP 6101/26
(Oesterreich)

## Patentansprüche

1. Fungizides Mittel, dadurch gekennzeichnet, dass es eine wirksame Menge mindestens einer Verbindung der allgemeinen Formel

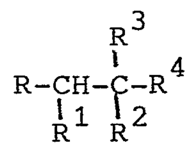

I

worin R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ gegebenenfalls mit 1 bis 3 Halogenatomen substituiertes $C_{3-6}$-Alkenyl, $C_{3-6}$-Alkinyl, $C_{3-6}$-Alkenyloxy, $C_{3-6}$-Alkinyloxy, $C_{3-6}$-Alkenylthio oder $C_{3-6}$-Alkinylthio; oder gegebenenfalls im Arylkern mono-, di- oder trisubstituiertes Aryl-$C_{1-3}$-alkyl, Aryloxy oder Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind,

$R^2$ Hydroxy, Chlor oder Brom,

$R^3$ Wasserstoff oder $C_{1-4}$-Alkyl

und $R^4$ 3-Pyridyl, 3-Pyridyl-1-oxid, 2-Pyrazinyl, 2-Pyrazinyl-1-oxid, 2-Pyrazinyl-4-oxid, 2-Pyrazinyl-1,4-dioxid, 5-Pyrimidinyl, 5-Pyrimidinyl-1-oxid oder 5-Pyrimidinyl-1,3-dioxid bedeuten,

oder eines Säureadditionssalzes einer solchen Verbindung, sowie Formulierungshilfsstoffe enthält.

2. Fungizides Mittel nach Anspruch 1, worin R 2,4-Dichlorphenyl bedeutet.

3. Fungizides Mittel nach Anspruch 1 oder 2, worin $R^1$ gegebenenfalls mit 1 bis 3 Halogenatomen substituiertes $C_{3-6}$-Alkenyl oder $C_{3-6}$-Alkinyl, oder gegebenenfalls mono-, di- oder trisubstituiertes Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind.

4. Fungizides Mittel nach einem der Ansprüche 1 bis 3, worin $R^3$ Wasserstoff oder Methyl bedeutet.

5. Fungizides Mittel nach einem der Ansprüche 1 bis 4, worin $R^4$ 3-Pyridyl, 2-Pyrazinyl oder 5-Pyrimidinyl bedeutet.

6. Fungizides Mittel nach Anspruch 1, dadurch gekennzeichnet, dass es eine wirksame Menge von α-(α-Allyl-2,4-dichlorbenzyl)-3-pyridylmethanol oder α-[2,4-Dichlor-α-(2-propinyl)-benzyl]-3-pyridylmethanol oder α-[α-(2,4-Dichlorphenyl)-phenäthyl]-3-pyridylmethanol oder α-(2,4-Dichlor-α-phenylthio-benzyl)-3-pyridylmethanol sowie Formulierungshilfsstoffe enthält.

7. Fungizides Mittel nach Anspruch 1, dadurch gekennzeichnet, dass es eine wirksame Menge einer Verbindung ausgewählt aus:

2-(2,4-Dichlorphenyl)-1,3-di-(3-pyridyl)-1-propanol,
α-[2,4-Dichlor-α-(2-methallyl)-benzyl]-3-pyridylmethanol,
α-(α-Allyl-2,4-dichlorbenzyl)-2-pyrazinylmethanol,
α-[2,4-Dichlor-α-(2-propinyl)-benzyl]-5-pyrimidinylmethanol,
α-[2,4-Dichlor-α-(4-chlorphenylthio)-benzyl]-3-pyridylmethanol,
α-[2,4-Dichlor-α-(2-pyridylthio)-benzyl]-3-pyridylmethanol,
α-[α-(2,3-Dibromallyl)-2,4-dichlorbenzyl]-3-pyridyl-

methanol,

2-(2,4-Dichlorphenyl)-3-(2-thienyl)-1-(3-pyridyl)-1-propanol,

α-[2,4-Dichlor-α-(2-furfuryl)-benzyl]-3-pyridylmethanol und

3-[1-Chlor-2-(2,4-dichlorphenyl)-4-pentenyl]-pyridin, sowie Formulierungshilfsstoffe enthält.

8. Fungizides Mittel nach Anspruch 1, dadurch gekennzeichnet, dass es eine wirksame Menge einer Verbindung ausgewählt aus:

α-[2,4-Dichlor-α-(3-methyl-2-butenyl)-benzyl]-3-pyridylmethanol,

α-[α-(2,4-Dichlorphenyl)-p-nitrophenäthyl]-3-pyridylmethanol,

α-[1-(2,4-Dichlorphenyl)-3-chlor-3-butenyl]-3-pyridylmethanol,

α-[1-(2,4-Dichlorphenyl)-4-chlor-3-butenyl]-3-pyridylmethanol,

α-(α-Allyl-2,4-dichlorbenzyl)-α-methyl-3-pyridylmethanol,

α-(α-Allyl-2,4-dichlorbenzyl)-α-äthyl-3-pyridylmethanol,

α-[2,4-Dichlor-α-(2-furfuryl)-benzyl]-α-methyl-3-pyridylmethanol,

3-(2,4-Dichlorphenyl)-4-(2-thienyl)-2-(3-pyridyl)-2-butanol,

α-[1-(2,4-Dichlorphenyl)-3-chlor-3-butenyl]-α-methyl-3-pyridylmethanol,

α-[1-(2,4-Dichlorphenyl)-4-chlor-3-butenyl]-α-methyl-3-pyridylmethanol und

α-[2,4-Dichlor-α-(2-thenyl)-benzyl]-α-methyl-3-pyridylmethanol-1-oxid,

sowie Formulierungshilfsstoffe enthält.

9. Verfahren zur Herstellung von Verbindungen der allgemeinen Formel

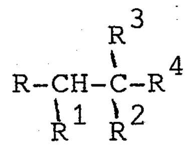

I

worin R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ gegebenenfalls mit 1 bis 3 Halogenatomen substituiertes $C_{3-6}$-Alkenyl, $C_{3-6}$-Alkinyl, $C_{3-6}$-Alkenyloxy, $C_{3-6}$-Alkinyloxy, $C_{3-6}$-Alkenylthio oder $C_{3-6}$-Alkinylthio; oder gegebenenfalls im Arylkern mono-, di- oder trisubstituiertes Aryl-$C_{1-3}$-alkyl, Aryloxy oder Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind,

$R^2$ Hydroxy, Chlor oder Brom,

$R^3$ Wasserstoff oder $C_{1-4}$-Alkyl

und $R^4$ 3-Pyridyl, 3-Pyridyl-1-oxid, 2-Pyrazinyl, 2-Pyrazinyl-1-oxid, 2-Pyrazinyl-4-oxid, 2-Pyrazinyl-1,4-dioxid, 5-Pyrimidinyl, 5-Pyrimidinyl-1-oxid oder 5-Pyrimidinyl-1,3-dioxid bedeuten,

und von ihren Säureadditionssalzen, dadurch gekennzeichnet, dass man

a) zwecks Herstellung der Verbindungen der Formel I, worin $R^1$ gegebenenfalls substituiertes $C_{3-6}$-Alkenyloxy, $C_{3-6}$-Alkinyloxy oder Aryloxy, wie oben näher definiert, $R^2$ Hydroxy und $R^4$ 3-Pyridyl, 2-Pyrazinyl oder 5-Pyrimidinyl bedeuten, ein Epoxid der allgemeinen Formel

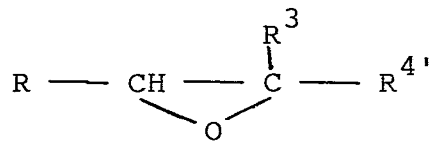 II

worin R und $R^3$ die oben angegebenen Bedeutungen besitzen

und $R^{4'}$ 3-Pyridyl, 2-Pyrazinyl oder 5-Pyrimidinyl bedeutet,

oder einen Alkohol der allgemeinen Formel

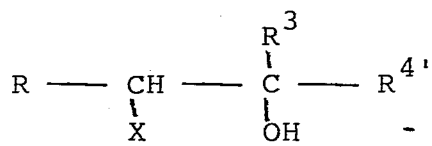 III

worin R, $R^3$ und $R^{4'}$ die oben angegebenen Bedeutungen besitzen

und X ein Halogenatom, einen Alkylsulfonatrest oder einen Arylsulfonatrest bedeutet,

mit einer Verbindung der allgemeinen Formel

$$R^{1'} — H \qquad IV$$

worin $R^{1'}$ gegebenenfalls mit 1 bis 3 Halogenatomen substituiertes $C_{3-6}$-Alkenyloxy oder $C_{3-6}$-Alkinyloxy; oder gegebenenfalls mono-, di- oder trisubstituiertes Aryloxy, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind, bedeutet,

umsetzt,

b) zwecks Herstellung der Verbindungen der Formel I, worin $R^1$ gegebenenfalls substituiertes $C_{3-6}$-Alkenyl, $C_{3-6}$-Alkinyl, $C_{3-6}$-Alkenylthio, $C_{3-6}$-Alkinylthio, Aryl-$C_{1-3}$-alkyl oder Arylthio, wie oben näher definiert, $R^2$ Hydroxy, $R^3$ Wasserstoff und $R^4$ 3-Pyridyl, 2-Pyrazinyl oder 5-Pyrimidinyl be-

DP 6103/26 0117485

deuten, ein Keton der allgemeinen Formel

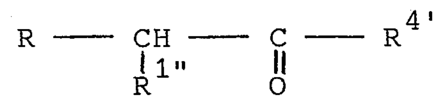
V

worin R und $R^{4'}$ die oben angegebenen Bedeutungen besitzen
und $R^{1''}$ gegebenenfalls mit 1 bis 3 Halogenatomen substituiertes $C_{3-6}$-Alkenyl, $C_{3-6}$-Alkinyl, $C_{3-6}$-Alkenylthio oder $C_{3-6}$-Alkinylthio; oder gegebenenfalls im Arylkern mono-, di- oder trisubstituiertes Aryl-$C_{1-3}$-alkyl oder Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind, bedeutet,

reduziert,

c) zwecks Herstellung der Verbindungen der Formel I, worin $R^1$ gegebenenfalls substituiertes $C_{3-6}$-Alkenyl, $C_{3-6}$-Alkinyl, $C_{3-6}$-Alkenylthio, $C_{3-6}$-Alkinylthio, Aryl-$C_{1-3}$-alkyl oder Arylthio, wie oben näher definiert, $R^2$ Hydroxy, $R^3$ $C_{1-4}$-Alkyl und $R^4$ 3-Pyridyl, 2-Pyrazinyl oder 5-Pyrimidinyl bedeuten, ein Keton der allgemeinen Formel V, wie oben definiert, mit einer Verbindung der allgemeinen Formel

$$R^{3'} — Y \qquad VI$$

worin $R^{3'}$ $C_{1-4}$-Alkyl,
Y Lithium oder MgZ
und Z Halogen bedeuten,

umsetzt,

d) zwecks Herstellung der Verbindungen der Formel I, worin $R^2$ Chlor oder Brom und $R^4$ 3-Pyridyl, 2-Pyrazinyl oder 5-Pyrimidinyl bedeuten, einen Alkohol der allgemeinen Formel

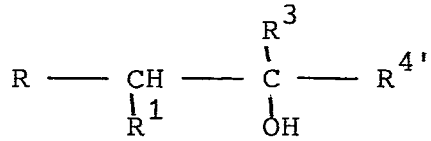

I'

worin R, $R^1$, $R^3$ und $R^{4'}$ die oben angegebenen Bedeutungen besitzen,

mit einem Chlorierungsmittel bzw. Bromierungsmittel behandelt,

e) zwecks Herstellung der Verbindungen der Formel I, worin $R^1$ gegebenenfalls substituiertes $C_{3-6}$-Alkenylthio, $C_{3-6}$-Alkinylthio oder Arylthio, wie oben näher definiert, und $R^2$ Hydroxy bedeuten, einen Thioäther der allgemeinen Formel

$$R — CH_2 — R^{1'''} \qquad VII$$

worin R die oben angegebene Bedeutung besitzt und $R^{1'''}$ gegebenenfalls mit 1 bis 3 Halogenatomen substituiertes $C_{3-6}$-Alkenylthio oder $C_{3-6}$-Alkinylthio; oder gegebenenfalls mono-, di- oder trisubstituiertes Arylthio, wobei die gegebenenfalls vorhandenen Substituenten 1 bis 3 Halogenatome und/oder 1 oder 2 $C_{1-3}$-Alkylgruppen und/oder 1 oder 2 $C_{1-3}$-Alkoxygruppen und/oder eine Nitrogruppe sind, bedeutet,

mit einem Aldehyd bzw. Keton der allgemeinen Formel

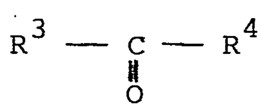

VIII

worin $R^3$ und $R^4$ die oben angegebenen Bedeutungen besitzen,

umsetzt, oder

f) zwecks Herstellung der Verbindungen der Formel I, worin $R^4$ 3-Pyridyl-1-oxid, 2-Pyrazinyl-1-oxid, 2-Pyrazinyl-4-oxid, 2-Pyrazinyl-1,4-dioxid, 5-Pyrimidinyl-1-oxid oder

5-Pyrimidinyl-1,3-dioxid bedeutet, ein Pyridin-, Pyrazin- bzw. Pyrimidinderivat der allgemeinen Formel

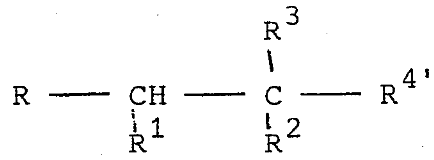

I"

worin R, $R^1$, $R^2$, $R^3$ und $R^{4'}$ die oben angegebenen Bedeutungen besitzen,

N-oxydiert

und erwünschtenfalls eine erhaltene Verbindung der Formel I mit einer Säure zur Herstellung des entsprechenden Säureadditionssalzes umsetzt.

10. Verfahren zur Herstellung eines fungiziden Mittels, dadurch gekennzeichnet, dass man mindestens eine der in Anspruch 1 genannten Verbindungen mit Formulierungshilfsstoffen vermischt.

11. Verfahren zur Bekämpfung von Fungi in der Landwirtschaft und im Gartenbau, dadurch gekennzeichnet, dass man das zu schützende Gut mit einer wirksamen Menge einer bzw. eines der in den Ansprüchen 1 bis 8 genannten Verbindungen bzw. Mittel behandelt.

12. Verwendung einer bzw. eines der in den Ansprüchen 1 bis 8 genannten Verbindungen bzw. Mittel zur Bekämpfung von Fungi in der Landwirtschaft und im Gartenbau.

***